# EUROPEAN PATENT SPECIFICATION

(11) **EP 2 491 134 B1**
(45) Date of publication and mention of the grant of the patent: **09.08.2017**
(21) Application number: 10768847.5
(22) Date of filing: 20.10.2010
(51) Int. Cl.: C12Q 1/68

(54) **GENETIC POLYMORPHISMS IN AGE-RELATED MACULAR DEGENERATION**
GENETISCHE POLYMORPHISMEN BEI ALTERSBEDINGTER MAKULADEGENERATION
POLYMORPHISME GÉNÉTIQUE DANS LA DÉGÉNÉRESCENCE MACULAIRE LIÉE À L'ÂGE

(30) Priority: 21.10.2009 US 253758 P
(43) Date of publication of application: 29.08.2012
(73) Proprietor: Genentech, Inc., South San Francisco, CA 94080 (US)
(72) Inventor: GRAHAM, Robert, South San Francisco California 94080 (US)
(74) Representative: Denison, Christopher Marcus
(86) International application number: PCT/US2010/053334
(87) International publication number: WO 2011/050034

(56) References cited:
- BRANTLEY ET AL: "Association of Complement Factor H and LOC387715 Genotypes with Response of Exudative Age-Related Macular Degeneration to Intravitreal Bevacizumab" OPHTHALMOLOGY, vol. 114, no. 12, 27 November 2007 (2007-11-27), pages 2168-2173, XP022382053 J. B. LIPPINCOTT CO., PHILADELPHIA, PA, US ISSN: 0161-6420 DOI: 10.1016/J.OPHTHA.2007.09.008
- LEE A Y ET AL: "Pharmacogenetics of complement factor H (Y402H) and treatment of exudative age-related macular degeneration with ranibizumab" BRITISH JOURNAL OF OPHTHALMOLOGY, vol. 93, no. 5, 1 May 2009 (2009-05-01), pages 610-613, XP009129122 LONDON, GB ISSN: 0007-1161
- DATABASE SNP [Online] 10 October 2003 (2003-10-10), XP002611532 retrieved from NCBI Database accession no. rs1064875
- DATABASE SNP [Online] 26 October 2003 (2003-10-26), XP002611533 retrieved from NCBI Database accession no. rs10917583
- DATABASE SNP [Online] 10 October 2003 (2003-10-10), XP002611534 retrieved from NCBI Database accession no. rs175714
- ENG K ET AL: "Ranibizumab in neovascular age-related macular degeneration" CLINICAL INTERVENTIONS IN AGING, vol. 1, no. 4, 1 January 2006 (2006-01-01), pages 451-466, XP007911685 DOVE MEDICAL PRESS, NZ ISSN: 1176-9092
- KLEIN ROBERT J ET AL: "Complement factor H polymorphism in age-related macular degeneration." SCIENCE (NEW YORK, N.Y.), vol. 308, no. 5720, 15 April 2005 (2005-04-15), pages 385-389, XP002611535 ISSN: 1095-9203
- ZHANG HONG ET AL: "The NEI/NCBI dbGAP database: Genotypes and haplotypes that may specifically predispose to risk of neovascular age-related macular degeneration" BMC MEDICAL GENETICS, vol. 9, June 2008 (2008-06), XP002611536 ISSN: 1471-2350

## Description

### FIELD OF THE INVENTION

This invention relates generally to treatment of human disease. More specifically, the invention relates to wet age-related macular degeneration (AMD).

### BACKGROUND OF THE INVENTION

AMD is a leading cause of severe, irreversible vision loss among the elderly. Bressler (2004) JAMA 291:1900-01. It is characterized by a broad spectrum of clinical and pathologic findings, including pale yellow spots known as drusen, disruption of the retinal pigment epithelium (RPE), choroidal neovascularization (CNV), and disciform macular degeneration. The disease is classified into two forms: non-exudative (dry) and exudative (wet or neovascular). Recently, several therapies have been developed for treatment of wet AMD - photodynamic therapy using verteporfin (Visudyne®); a VEGF-binding aptamer, pegaptanib (Macugen®); and an anti-VEGF antibody fragment, ranibizumab (Lucentis®).

Genetic polymorphisms occur in a population when different alleles in particular genes result in different phenotypes, including disease development or progression and responsiveness to therapeutic drugs. Multiple polymorphisms have been identified that are associated with development or progression of AMD *(e.g.,* Despriet et al. (2007) Arch. Ophthalmol. 125:1270-71; Seddon et al. (2007) JAMA 297:1793-99, 2585; Boon et al. (2008) Am. J. Human Genet. 82:516-23). Previous work has shown that particular polymorphisms at amino acid position 402 of the complement factor H *(CFH)* gene are associated with response to PDT with verteporfin or off-label bevacizumab therapy for AMD (Brantley et al. (2008) Eye published online 22 February, pp. 1-6; Brantley et al. (2007) Ophthalmology 114:2168-73). Identification of additional polymorphisms associated with development of disease and/or predictive of the efficacy or safety of particular therapies may be used to tailor therapies to those patients who would best benefit from them.

### SUMMARY OF THE INVENTION

The present invention is based in part on the identification of genetic polymorphisms that are predictive of AMD risk or an increased likelihood that treatment with high-affinity anti-VEGF antibodies will benefit patients with AMD.

In one aspect, the invention provides a method of predicting whether a wet AMD patient has an increased likelihood of benefiting from treatment with a high-affinity anti-VEGF antibody, comprising screening a sample isolated from said patient for a genomic polymorphism in the matrix metalloprotease 25 gene (MMP25) allele corresponding to rs1064875, wherein the patient has an increased likelihood of benefiting from said treatment if the corresponding genotype comprises AA or AG. In some embodiments, the genotype comprises AA. In some embodiments, the genotype comprises AG.

In another aspect, the invention provides a method of predicting whether a wet AMD patient has an increased likelihood of benefiting from treatment with an anti-VEGF antibody, comprising screening a sample isolated from said patient for a genomic polymorphism in the basic leucine zipper transcription factor, ATF-like (BATF) allele corresponding to rs175714, wherein the patient has an increased likelihood of benefiting from said treatment if the corresponding genotype comprises AA or AG. In some embodiments, the genotype comprises AA. In some embodiments, the genotype comprises AG.

In some embodiments, the anti-VEGF antibody binds the same epitope as the monoclonal anti-VEGF antibody A4.6.1 produced by hybridoma ATCC® HB 10709. In some embodiments, the anti-VEGF antibody has a heavy chain variable domain comprising the following heavy chain complementarity determining region (CDR) amino acid sequences: CDRH1 (GYDFTHYGMN; SEQ ID NO: 1), CDRH2 (WINTYTGEPTYAADFKR; SEQ ID NO: 2) and CDRH3 (YPYYYGTSHWYFDV; SEQ ID NO: 3) and a light chain variable domain comprising the following light chain CDR amino acid sequences: CDRL1 (SASQDISNYLN; SEQ ID NO: 4), CDRL2 (FTSSLHS; SEQ ID NO: 5) and CDRL3 (QQYSTVPWT; SEQ ID NO: 6). In some embodiments, the anti-VEGF antibody has the heavy chain variable domain and light chain variable domain of Y0317. In some embodiments, the anti-VEGF antibody is ranibizumab.

Also disclosed is a kit for predicting whether a wet AMD patient has an increased likelihood of benefiting from treatment with ranibizumab comprising a first oligonucleotide and a second oligonucleotides specific for an A/G polymorphism in the MMP25 allele corresponding to rs1064875. In some embodiments, the first oligonucleotide and said second oligonucleotide may be used to amplify a part of the MMP25 gene comprising an A/G polymorphism in the MMP25 allele corresponding to rs1064875.

Also disclosed is a kit for predicting whether a wet AMD patient has an increased likelihood of benefiting from treatment with ranibizumab comprising a first oligonucleotide and a second oligonucleotides specific for an A/G polymorphism in the BATF allele corresponding to rs175714. In some embodiments, the first oligonucleotide and said second oligonucleotide may be used to amplify a part of the BATF gene comprising an A/G polymorphism in the BATF allele corresponding to rs175714.

Also disclosed are methods for determining whether a patient is at increased risk of developing wet AMD comprising screening a sample isolated from the patient for one or more of the allelic variants shown in Table 4 or Table 5, wherein the presence of one or more of the allelic variants shown in Table 4 or Table 5 indicates that the patient is at increased risk of developing wet AMD.

### BRIEF DESCRIPTION OF THE DRAWINGS

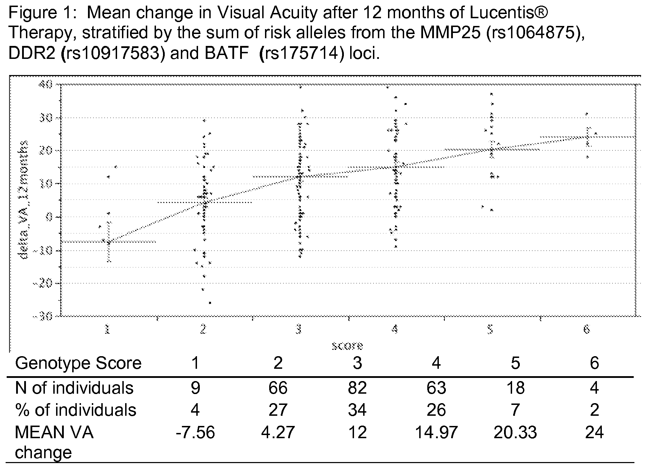

**FIGURE 1** shows the mean change in Visual Acuity after 12 months of Lucentis therapy stratified by the sum of risk alleles from the MMP25, DDR2, and BATF genes.

### DETAILED DESCRIPTION OF THE PREFERRED EMBODIMENTS

The practice of the present invention will employ, unless otherwise indicated, conventional techniques of molecular biology (including recombinant techniques), microbiology, cell biology, biochemistry, and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Current Protocols in Molecular Biology" (F. M. Ausubel et al., eds., 1987, and periodic updates); "PCR: The Polymerase Chain Reaction", (Mullis et al., eds., 1994).

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application.

### DEFINITIONS

As used herein, the singular forms "a", "an" and "the" include the plural unless the context clearly dictates otherwise. For example, "a" cell will also include "cells".

The term "comprising" is intended to mean that the compositions and methods include the recited elements, but do not exclude others.

The terms "VEGF" and "VEGF-A" are used interchangeably to refer to the 165-amino acid vascular endothelial cell growth factor and/or related 121-, 189-, and 206- amino acid vascular endothelial cell growth factors, as described by Leung et al. Science, 246:1306 (1989), and Houck et al. Mol. Endocrin., 5:1806 (1991), together with the naturally occurring allelic and processed forms thereof.

An "anti-VEGF antibody" is an antibody that binds to VEGF with sufficient affinity and specificity. Preferably, the anti-VEGF antibody of the invention can be used as a therapeutic agent in targeting and interfering with diseases or conditions wherein the VEGF activity is involved. An anti-VEGF antibody will usually not bind to other VEGF homologues such as VEGF-B or VEGF-C, or other growth factors such as P1GF, PDGF or bFGF. A preferred anti-VEGF antibody is a monoclonal antibody that binds to the same epitope as the monoclonal anti-VEGF antibody A4.6.1 produced by hybridoma ATCC® HB 10709 and is a high-affinity anti-VEGF antibody. A "high-affinity anti-VEGF antibody" has at 1cast 10-fold better affinity for VEGF than the monoclonal anti-VEGF antibody A4.6.1. Preferably the anti-VEGF antibody is a recombinant humanized anti-VEGF monoclonal antibody fragment generated according to WO 98/45331, including an antibody comprising the CDRs and/or the variable regions of Y0317. More preferably, the anti-VEGF antibody is the antibody fragment known as ranibizumab (Lucentis®)

The term "antibody" is used in the broadest sense and includes monoclonal antibodies (including full length or intact monoclonal antibodies), polyclonal antibodies, multivalent antibodies, multispccific antibodies *(e.g.,* bispccific antibodies), and antibody fragments so long as they exhibit the desired biological activity.

"Treatment" refers to both therapeutic treatment and prophylactic or preventative measures. Those in need of treatment include those already with the disorder as well as those in which the disorder is to be prevented or delayed.

The term "polymorphism" refers to a location in the sequence of a gene which varies within a population. A polymorphism is comprised of different "alleles". The location of such a polymorphism may be identified by its position in the gene and the different amino acids or bases that are found there. For example, *Y402H CFH* indicates that there is variation between tyrosine (Y) and histidine (H) at amino acid position 402 in the *CFH* gene. This amino acid change is the result of two possible variant bases, C and T, which are two different alleles. Because the genotype is comprised of two separate alleles, any of several possible variants may be observed in any one individual *(e.g.* for this example, CC, CT, or TT). Individual polymorphisms are also assigned unique identifiers ("Reference SNP", "refSNP" or "rs#") known to one of skill in the art and used, *e.g.,* in the Single Nucleotide Polymorphism Database (dbSNP) of Nucleotide Sequence Variation available on the NCBI website.

The term "genotype" refers to the specific alleles of a certain gene in a cell or tissue sample. In the example above, CC, CT, or TT are possible genotypes at the *Y402H CFH* polymorphism.

The term "sample" includes a cell or tissue sample taken from a patient. For example, a sample may include a skin sample, a cheek cell sample, or blood cells.

Identification of the particular genotype in a sample may be performed by any of a number of methods well known to one of skill in the art. For example, identification of the polymorphism can be accomplished by cloning of the allele and sequencing it using techniques well known in the art. Alternatively, the gene sequences can be amplified from genomic DNA, *e.g.* using PCR, and the product sequenced. Several non-limiting methods for analyzing a patient's DNA for mutations at a given genetic locus are described below.

DNA microarray technology, *e.g.,* DNA chip devices and high-density microarrays for high-throughput screening applications and lower-density microarrays, may be used. Methods for microarray fabrication are known in the art and include various inkjet and microjet deposition or spotting technologies and processes, *in situ* or on-chip photolithographic oligonucleotide synthesis processes, and electronic DNA probe addressing processes. The DNA microarray hybridization applications has been successfully applied in the areas of gene expression analysis and genotyping for point mutations, single nucleotide polymorphisms (SNPs), and short tandem repeats (STRs). Additional methods include interference RNA microarrays and combinations of microarrays and other methods such as laser capture microdissection (LCM), comparative genomic hybridization (CGH) and chromatin immunoprecipitation (ChiP). See, *e.g.,* He et al. (2007) Adv. Exp. Med. Biol. 593:117-133 and Heller (2002) Annu. Rev. Biomed. Eng. 4:129-153. Other methods include PCR, xMAP, invader assay, mass spectrometry, and pyrosequencing (Wang et al. (2007) Microarray Technology and Cancer Gene Profiling Vol 593 of book series Advances in Experimental Medicine and Biology, pub. Springer New York).

Another detection method is allele specific hybridization using probes overlapping the polymorphic site and having about 5, or alternatively 10, or alternatively 20, or alternatively 25, or alternatively 30 nucleotides around the polymorphic region. For example, several probes capable of hybridizing specifically to the allelic variant are attached to a solid phase support, e.g., a "chip". Oligonucleotides can be bound to a solid support by a variety of processes, including lithography. Mutation detection analysis using these chips comprising oligonucleotides, also termed "DNA probe arrays" is described e.g., in Cronin et al. (1996) Human Mutation 7:244.

In other detection methods, it is necessary to first amplify at least a portion of the gene prior to identifying the allelic variant. Amplification can be performed, e.g., by PCR and/or LCR or other methods well known in the art.

In some cases, the presence of the specific allele in DNA from a subject can be shown by restriction enzyme analysis. For example, the specific nucleotide polymorphism can result in a nucleotide sequence comprising a restriction site which is absent from the nucleotide sequence of another allelic variant.

In a further embodiment, protection from cleavage agents (such as a nuclease, hydroxylamine or osmium tetroxide and with piperidine) can be used to detect mismatched bases in RNA/RNA DNA/DNA, or RNA/DNA heteroduplexes *(see, e.g.,* Myers et al. (1985) Science 230:1242). In general, the technique of "mismatch cleavage" starts by providing heteroduplexes formed by hybridizing a control nucleic acid, which is optionally labeled, *e.g.,* RNA or DNA, comprising a nucleotide sequence of the allelic variant of the gene with a sample nucleic acid, *e.g.,* RNA or DNA, obtained from a tissue sample. The double-stranded duplexes are treated with an agent which cleaves single-stranded regions of the duplex such as duplexes formed based on basepair mismatches between the control and sample strands. For instance, RNA/DNA duplexes can be treated with RNase and DNA/DNA hybrids treated with S 1 nuclease to enzymatically digest the mismatched regions. Alternatively, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine whether the control and sample nucleic acids have an identical nucleotide sequence or in which nucleotides they are different. See, for example, U.S. Pat. No. 6,455,249; Cotton et al. (1988) Proc. Natl. Acad. Sci. USA 85:4397; Saleeba et al. (1992) Meth. Enzymol. 217:286-295.

Alterations in electrophoretic mobility may also be used to identify the particular allelic variant. For example, single strand conformation polymorphism (SSCP) may be used to detect differences in electrophoretic mobility between mutant and wild type nucleic acids (Orita et al. (1989) Proc Natl. Acad. Sci USA 86:2766; Cotton (1993) Mutat. Res. 285:125-144 and Hayashi (1992) Genet. Anal. Tech. Appl. 9:73-79). Single-stranded DNA fragments of sample and control nucleic acids are denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In another preferred embodiment, the subject method utilizes heteroduplex analysis to separate double stranded heteroduplex molecules on the basis of changes in electrophoretic mobility (Keen et al. (1991) Trends Genet. 7:5*).*

The identity of the allelic variant may also be obtained by analyzing the movement of a nucleic acid comprising the polymorphic region in polyacrylamide gels containing a gradient of denaturant, which is assayed using denaturing gradient gel electrophoresis (DGGE) (Myers et al. (1985) Nature 313:495). When DGGE is used as the method of analysis, DNA will be modified to ensure that it does not completely denature, for example by adding a GC clamp of approximately 40 bp of high-melting GC-rich DNA by PCR. In a further embodiment, a temperature gradient is used in place of a denaturing agent gradient to identify differences in the mobility of control and sample DNA (Rosenbaum and Reissner (1987) Biophys. Chem. 265:1275).

Examples of techniques for detecting differences of at least one nucleotide between 2 nucleic acids include, but are not limited to, selective oligonucleotide hybridization, selective amplification, or selective primer extension. For example, oligonucleotide probes may be prepared in which the known polymorphic nucleotide is placed centrally (allele-specific probes) and then hybridized to target DNA under conditions which permit hybridization only if a perfect match is found (Saiki et al. (1986) Nature 324:163); Saiki et al. (1989) Proc. Natl. Acad. Sci. USA 86:6230). Such allele specific oligonucleotide hybridization techniques may be used for the detection of the nucleotide changes in the polymorphic region of the gene. For example, oligonucleotides having the nucleotide sequence of the specific allelic variant are attached to a hybridizing membrane and this membrane is then hybridized with labeled sample nucleic acid. Analysis of the hybridization signal will then reveal the identity of the nucleotides of the sample nucleic acid.

Alternatively, allele specific amplification technology which depends on selective PCR amplification may be used in conjunction with the instant invention. Oligonucleotides used as primers for specific amplification may carry the allelic variant of interest in the center of the molecule (so that amplification depends on differential hybridization) (Gibbs et al. (1989) Nucl. Acids Res. 17:2437-2448) or at the extreme 3' end of one primer where, under appropriate conditions, mismatch can prevent, or reduce polymerase extension (Prossner (1993) Tibtech 11:238 and Newton et al. (1989) Nucl. Acids Res. 17:2503). This technique is also termed "PROBE" for PRobc Oligo Base Extension. In addition it may be desirable to introduce a novel restriction site in the region of the mutation to create cleavage-based detection (Gasparini et al. (1992) Mol. Cell. Probes 6:1).

In another embodiment, identification of the allelic variant is carried out using an oligonucleotide ligation assay (OLA), as described, *e.g.,* in U.S. Pat. No. 4,998,617 and in Laridegren, U. et al. Science 241:1077-1080 (1988). The OLA protocol uses two oligonucleotides which are designed to be capable of hybridizing to abutting sequences of a single strand of a target. One of the oligonucleotides is linked to a separation marker, e.g., biotinylated, and the other is detectably labeled, If the precise complementary sequence is found in a target molecule, the oligonucleotides will hybridize such that their termini abut, and create a ligation substrate. Ligation then permits the labeled oligonucleotide to be recovered using avidin, or another biotin ligand. Nickerson, D. A. et al. have described a nucleic acid detection assay that combines attributes of PCR and OLA (Nickerson, D. A. et al. (1990) Proc. Natl. Acad. Sci. USA 87:8923-8927). In this method, PCR is used to achieve the exponential amplification of target DNA, which is then detected using OLA.

The invention provides methods as defined in the claims, in which a single nucleotide polymorphism (SNP) in MMP25 or BATF is detected. Because single nucleotide polymorphisms are flanked by regions of invariant sequence, their analysis requires no more than the determination of the identity of the single variant nucleotide and it is unnecessary to determine a complete gene sequence for each patient. Several methods have been developed to facilitate the analysis of SNPs.

The single base polymorphism can be detected by using a specialized exonuclease-resistant nucleotide, as disclosed, *e.g.,* in U.S. Pat. No. 4,656,127. According to the method, a primer complementary to the allelic sequence immediately 3' to the polymorphic site is permitted to hybridize to a target molecule obtained from a particular animal or human. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identity of the exonuclease-resistant derivative of the sample is known, a finding that the primer has become resistant to exonucleases reveals that the nucleotide present in the polymorphic site of the target molecule was complementary to that of the nucleotide derivative used in the reaction. This method has the advantage that it docs not require the determination of large amounts of extraneous sequence data.

A solution-based method may also be used for determining the identity of the nucleotide of the polymorphic site (WO 91/02087). As above, a primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

An alternative method is described in WO 92/15712. This method uses mixtures of labeled terminators and a primer that is complementary to the sequence 3' to a polymorphic site. The labeled terminator that is incorporated is thus determined by, and complementary to, the nucleotide present in the polymorphic site of the target molecule being evaluated. The method is usually a heterogeneous phase assay, in which the primer or the target molecule is immobilized to a solid phase.

Many other primer-guided nucleotide incorporation procedures for assaying polymorphic sites in DNA have been described (Komher, J. S. et al. (1989) Nucl. Acids. Res. 17:7779-7784; Sokolov, B. P. (1990) Nucl. Acids Res. 18:3671; Syvanen, A.-C., et al. (1990) Genomics 8:684-692; Kuppuswamy, M. N. et al. (1991) Proc. Natl. Acad. Sci. USA 88:1143-1147; Prezant, T. R. et al. (1992) Hum. Mutat. 1: 159-164; Ugozzoli, L. et al. (1992) GATA 9:107-112; Nyren, P. et al. (1993) Anal. Biochem. 208:171-175). These methods all rely on the incorporation of labeled deoxynucleotides to discriminate between bases at a polymorphic site.

Moreover, it will be understood that any of the above methods for detecting alterations in a gene or gene product or polymorphic variants can be used to monitor the course of treatment or therapy.

The methods described herein may be performed, for example, by utilizing prepackaged diagnostic kits, such as those described below, comprising at least one probe or primer nucleic acid, which may be conveniently used, *e.g.,* to determine whether an individual has an increased likelihood of developing AMD or whether a wet AMD patient has an increased likelihood of benefiting from treatment with an anti-VEGF antibody.

Sample nucleic acid for use in the above-described diagnostic and prognostic methods can be obtained from any cell type or tissue of a subject. For example, a subject's bodily fluid (e.g. blood) can be obtained by known techniques. Alternatively, nucleic acid tests can be performed on dry samples *(e.g.,* hair or skin).

The invention described herein relates to methods as defined in the claims, which include determining and identifying the allele present at the MMP25 or BATF alleles at rs1064875 and rs175714, respectively. Probes can be used to directly determine the genotype of the sample or can be used simultaneously with or subsequent to amplification. The term "probes" includes naturally occurring or recombinant single- or double-stranded nucleic acids or chemically synthesized nucleic acids. They may be labeled by nick translation, Klenow fill-in reaction, PCR or other methods known in the art. Probes,

their preparation and/or labeling arc described in Sambrook ct al. (1989) *supra.* A probe can be a polynucleotide of any length suitable for selective hybridization to a nucleic acid containing a polymorphic region of the invention. Length of the probe used will depend, in part, on the nature of the assay used and the hybridization conditions employed.

Labeled probes also can be used in conjunction with amplification of a polymorphism. (Holland et al. (1991) Proc. Natl. Acad. Sci. USA 88:7276-7280). U.S. Pat. No. 5,210,015 describes fluorescence-based approaches to provide real time measurements of amplification products during PCR. Such approaches have either employed intercalating dyes (such as ethidium bromide) to indicate the amount of double-stranded DNA present, or they have employed probes containing fluorescence-quencher pairs (also referred to as the "TaqMan®" approach) where the probe is cleaved during amplification to release a fluorescent molecule whose concentration is proportional to the amount of double-stranded DNA present. During amplification, the probe is digested by the nuclease activity of a polymerase when hybridized to the target sequence to cause the fluorescent molecule to be separated from the quencher molecule, thereby causing fluorescence from the reporter molecule to appear. The TaqMan® approach uses a probe containing a reporter molecule--quencher molecule pair that specifically anneals to a region of a target polynucleotide containing the polymorphism.

Probes can be affixed to surfaces for use as "gene chips." Such gene chips can be used to detect genetic variations by a number of techniques known to one of skill in the art. In one technique, oligonucleotides are arrayed on a gene chip for determining the DNA sequence of a by the sequencing by hybridization approach, such as that outlined in U.S. Pat. Nos. 6,025,136 and 6,018,041. The probes also can be used for fluorescent detection of a genetic sequence. Such techniques have been described, for example, in U.S. Pat. Nos. 5,968,740 and 5,858,659. A probe also can be affixed to an electrode surface for the electrochemical detection of nucleic acid sequences such as described in U.S. Pat. No. 5,952,172 and by Kelley, S. O. et al. (1999) Nucl. Acids Res. 27:4830-4837.

Additionally, the isolated nucleic acids used as probes or primers may be modified to become more stable. Exemplary nucleic acid molecules which arc modified include phosphoramidate, phosphothioate and methylphosphonate analogs of DNA (see also U.S. Pat. Nos. 5,176,996; 5,264,564 and 5,256,775).

As set forth herein, the invention provides methods, as defined in the claims, which include determining the type of allelic variants of polymorphic regions present in MMP25 or BATF. In some embodiments, the methods use probes or primers comprising nucleotide sequences which arc complementary to a polymorphic region of MMP25 or BATF. Accordingly, kits for performing these methods are also disclosed.

A kit for determining whether a wet AMD patient has an increased likelihood of benefiting from treatment with an anti-VEGF antibody, including a high-affinity anti-VEGF antibody contains one of more of the compositions described herein and instructions for use. As an example only, a kit for determining whether a wet AMD patient has an increased likelihood of benefiting from treatment with ranibizumab comprises a first oligonucleotide and a second oligonucleotides specific for a AG polymorphism in the MMP25 rs1064875 SNP. Oligonucleotides "specific for" a genetic locus bind either to the polymorphic region of the locus or bind adjacent to the polymorphic region of the locus. For oligonucleotides that are to be used as primers for amplification, primers are adjacent if they are sufficiently close to be used to produce a polynucleotide comprising the polymorphic region. In one embodiment, oligonucleotides are adjacent if they bind within about 1-2 kb, *e.g.* less than 1 kb from the polymorphism. Specific oligonucleotides are capable of hybridizing to a sequence, and under suitable conditions will not bind to a sequence differing by a single nucleotide.

The kit can comprise at least one probe or primer which is capable of specifically hybridizing to the polymorphic region of MMP25 or BATF and instructions for use. The kits usually comprise at least one of the above described nucleic acids. Kits for amplifying at least a portion of MMP25 or BATF generally comprise two primers, at least one of which is capable of hybridizing to the allelic variant sequence. Such kits are suitable for detection of genotype by, for example, fluorescence detection, by electrochemical detection, or by other detection.

Oligonucleotides, whether used as probes or primers, contained in a kit can be detectably labeled. Labels can be detected either directly, for example for fluorescent labels, or indirectly. Indirect detection can include any detection method known to one of skill in the art, including biotin-avidin interactions, antibody binding and the like. Fluorescently labeled oligonucleotides also can contain a quenching molecule. Oligonucleotides can be bound to a surface. In some embodiments, the surface is silica or glass. In some embodiments, the surface is a metal electrode.

Yet other kits comprise at least one reagent necessary to perform the assay. For example, the kit can comprise an enzyme. Alternatively the kit can comprise a buffer or any other necessary reagent.

The kits can include all or some of the positive controls, negative controls, reagents, primers, sequencing markers, probes and antibodies described herein for determining the subject's genotype in the polymorphic region of MMP25, DDR2 or BATF.

The following example is intended merely to illustrate the practice of the present invention and is not provided by way of limitation.

### EXAMPLE

### Example 1. Genetic polymorphisms and their association with AMD occurrence and treatment outcomes

### Samples and genotyping

Peripheral blood samples from 250 de-identified subjects from Lucentis® pivotal trials (MARINA, ANCHOR, and FOCUS) who participated in the DAWN genetic substudy of the HORIZON extension trial were collected and genomic DNA was isolated. All samples used in the analysis had a self-identified race listed as "White" and had a confirmed diagnosis of neo-vascular AMD. The samples consisted of 104 males and 146 females, and the average age at baseline was 75.7 years of age. Written informed consent was obtained from all individuals in the study and the study protocols were approved by institutional review boards.

In addition to the samples described above, 102 samples were collected as part of the DAWN study, resulting in a total of 352 total samples. The 352 samples were genotyped using the Illumina® 550K Human HapMap Bead Array. We used stringent quality control (QC) criteria to ensure that high quality data was included in the final analysis. Specifically, we a) excluded individuals who had > 5% missing data and b) excluded individuals based on cryptic relatedness and duplicate samples based on IBS status (PI_Hat > 0.15, none detected in this data set). We included only SNPs with a) < 5 % missing data, b) HWE p-value > 1x10⁻⁶, and c) MAF > 0.01 %. All QC tests were performed using PLINK (Purcell et al. (2007) Am. J. Hum. Genet. 81, 559-75).

### Genome-wide association scan for response to Lucentis® therapy

The DAWN samples were separated into 2 groups based on the treatment status during the MARINA, ANCHOR and FOCUS trials. The Lucentis® treated group included individuals who received doses of 0.3mg, 0.5 mg or 0.5mg+PDT (N=242). The SHAM/PDT group consisted of individuals who received a mock injection (SHAM) or only photodynamic therapy (PDT) (N=110). We performed a genome-wide association scan to identify genetic variants significantly associated with change in visual acuity (VA, measured in letters) after 12 months of Lucentis® treatment (Table 1). From the list of top loci associated with mean change in VA, we identified 3 loci that contain genes that are likely to play a role in wound repair (Table 2) and created a "gene score" by summing the number of risk alleles carried by each individual from the MMP25 (rs1064875), DDR2 (rs10917583) and BATF (rs175714) loci (Figure 1).

**Table 1: Rank ordered list of variants most associated with change in Visual Acuity after 12 months of Lucentis® therapy (P<2 x10⁻⁵).**

| Chromosome | SNP | Chromosomal Position (BP, based on NCBI build 36) | Allele | P value | Gene symbol |
|---|---|---|---|---|---|
| 1 | rs10917583 | 160897518 | G | 5.81E-06 | DDR2 |
| 22 | rs956548 | 25867502 | A | 7.10E-06 | MIAT |
| 1 | rs10494373 | 160885986 | C | 7.98E-06 | DDR2 |
| 19 | rs7260544 | 51313304 | C | 8.24E-06 | IGFL3 |
| 22 | rs9608580 | 25862474 | G | 9.70E-06 | MIAT |
| 10 | rs7913098 | 32014665 | A | 1.22E-05 | LOC646034 |
| 1 | rs16843630 | 160936667 | G | 1.27E-05 | DDR2 |
| 14 | rs175714 | 75051609 | A | 1.63E-05 | BATF |
| 16 | rs1064875 | 3042210 | A | 1.68E-05 | MMP25 |
| 1 | rs1910339 | 174640705 | A | 1.69E-05 | PAPPA2 |

**Table 2: Mean change in Visual Acuity at 12 months of Lucentis® treatment stratified by genotype at MMP25, DDR2 and BATF loci.**

| MMP25 (rs1064875) | N | % of population | Mean change in VA at 12 months |
|---|---|---|---|
| G/G | 153 | 63% | 8.4 |
| A/G | 72 | 30% | 13.1 |
| A/A | 16 | 7% | 22.4 |
| | | | |

| DDR2 (rs10917583) | N | % of population | Mean change in VA at 12 months |
|---|---|---|---|
| C/C | 2 | 1% | -22.0 |
| C/A | 35 | 15% | 4.2 |
| A/A | 204 | 85% | 12.3 |
| | | | |

| BATF (rs175714) | N | % of population | Mean change in VA at 12 months |
|---|---|---|---|
| G/G | 81 | 34% | 5.4 |
| A/G | 118 | 49% | 13.0 |
| A/A | 42 | 17% | 15.2 |

### Genome-wide association scan for Baseline clinical phenotypes

In all 352 DAWN samples we performed a genome-wide test for association to several baseline phenotypes, including: visual acuity at baseline (Table 3), Presence of CNV in the untreated Fellow eye (Table 4), and CNV classification (Predominantly classic vs minimally classic and occult classification, Table 5).

**Table 3: Rank ordered list of variants associated with change in Visual Acuity at baseline in 352 individuals (P<2 x10⁻⁵).**

| Chromosome | SNP | Chromosomal Position (BP, based on NCBI build 36) | Allele | P value | Permuted P value |
|---|---|---|---|---|---|
| 2 | rs707097 | 155027061 | G | 3.65E-07 | 0.00999 |
| 6 | rs9295181 | 162401450 | G | 1.82E-06 | 0.05295 |
| 9 | rs16927388 | 126159263 | A | 2.32E-06 | 0.05295 |
| 15 | rs4316697 | 47517217 | A | 9.50E-06 | 0.1239 |
| 10 | rs913035 | 29858610 | C | 1.04E-05 | 0.2218 |

**Table 4: Rank ordered list of variants associated with presence of CNV in the fellow eye at baseline in 352 individuals (P<2 x10⁻⁵).**

| Chromosome | SNP | Chromosomal Position (BP, based on NCBI build 36) | Allele | P value | Permuted P value | Gene symbol |
|---|---|---|---|---|---|---|
| 8 | rs2022976 | 107470989 | A | 1.20E-06 | 0.02498 | OXR1 |
| 9 | rs10813420 | 31013908 | A | 1.23E-06 | 0.02597 | LOC646753 |
| 17 | rs7210510 | 24495724 | A | 2.27E-06 | 0.02098 | MYO18A |
| 8 | rs7828669 | 124007968 | A | 6.22E-06 | 0.1159 | ZHX2 |
| 7 | rs11764261 | 180194 | A | 8.15E-06 | 0.1568 | LOC645561 |
| 1 | rs12741645 | 44335934 | A | 1.00E-05 | 0.2318 | LOC644743 |
| 7 | rs2082744 | 99319352 | G | 1.03E-05 | 0.2008 | TRIM4 |
| 9 | rs10970046 | 30993452 | G | 1.08E-05 | 0.1828 | LOC646753 |
| 8 | rs6991239 | 124006174 | G | 1.20E-05 | 0.2108 | ZHX2 |
| 8 | rs7819862 | 123993632 | G | 1.21E-05 | 0.2108 | ZHX2 |
| 14 | rs2144064 | 101188372 | G | 1.52E-05 | 0.1718 | C14orf72 |
| 7 | rs2571997 | 99352353 | A | 1.52E-05 | 0.2617 | TRIM4 |
| 7 | rs2572009 | 99326941 | A | 1.52E-05 | 0.2617 | TRIM4 |
| 12 | rs7297415 | 111145487 | A | 1.63E-05 | 0.2408 | NULL |
| 8 | rs1368137 | 130401289 | C | 1.63E-05 | 0.2577 | CCDC26 |
| 17 | rs4986765 | 57118247 | A | 1.65E-05 | 0.1528 | BRIP1 |
| 2 | rs1344759 | 122755725 | A | 1.77E-05 | 0.3986 | LOC728241 |

**Table 5: Rank ordered list of variants associated with CNV classification at baseline in 352 individuals (P<2 x10⁻⁵).**

| Chromosome | SNP | Chromosomal Position (BP, based on NCBI build 36) | Allele | P value | Permuted P value | Gene symbol |
|---|---|---|---|---|---|---|
| 10 | rs617738 | 84312795 | A | 3.53E-07 | 0.01199 | NRG3 |
| 6 | rs2076169 | 35496457 | G | 2.79E-06 | 0.06194 | PPARD |
| 10 | rs6480533 | 73022225 | G | 3.73E-06 | 0.08192 | CDH23 |
| 10 | rs594612 | 84257806 | G | 4.77E-06 | 0.1009 | NRG3 |
| 2 | rs13029532 | 191584146 | C | 1.12E-05 | 0.2428 | STAT1 |
| 10 | rs11819553 | 73096471 | G | 1.48E-05 | 0.2567 | CDH23 |
| 20 | rs1291117 | 34935654 | G | 1.62E-05 | 0.1508 | C20orf118 |
| 6 | rs726281 | 152344271 | G | 1.87E-05 | 0.3357 | ESR1 |

## Claims

1. A method of predicting whether a wet AMD patient has an increased likelihood of benefiting from treatment with a high-affinity anti-VEGF antibody, comprising screening a sample isolated from said patient for a genomic polymorphism in the matrix metalloprotease 25 gene (MMP25) allele corresponding to rs1064875, wherein the patient has an increased likelihood of benefiting from said treatment if the corresponding genotype comprises AA or AG.

2. A method of predicting whether a wet AMD patient has an increased likelihood of benefiting from treatment with an anti-VEGF antibody, comprising screening a sample isolated from said patient for a genomic polymorphism in the basic leucine zipper transcription factor, ATF-like (BATF) allele corresponding to rs175714, wherein the patient has an increased likelihood of benefiting from said treatment if the corresponding genotype comprises AA or AG.

3. The method of any one of claims 1 or 2, wherein said anti-VEGF antibody binds the same epitope as the monoclonal anti-VEGF antibody A4.6.1 produced by hybridoma ATCC® HB 10709.

4. The method of claim 3, wherein said anti-VEGF antibody has a heavy chain variable domain comprising the following heavy chain complementarity determining region (CDR) amino acid sequences: CDRH1 (GYDFTHYGMN; SEQ ID NO: 1), CDRH2 (WINTYTGEPTYAADFKR; SEQ ID NO: 2) and CDRH3 (YPYYYGTSHWYFDV; SEQ ID NO: 3) and a light chain variable domain comprising the following light chain CDR amino acid sequences: CDRL1 (SASQDISNYLN; SEQ ID NO: 4), CDRL2 (FTSSLHS; SEQ ID NO: 5) and CDRL3 (QQYSTVPWT; SEQ ID NO: 6).

5. The method of claim 4, wherein said anti-VEGF antibody has the heavy chain variable domain and light chain variable domain of Y0317.

6. The method of any one of claims 1 to 3, wherein said anti-VEGF antibody is ranibizumab.

7. The method of claim 1, wherein the corresponding genotype comprises AA.

8. The method of claim 1, wherein the corresponding genotype comprises AG.

9. The method of claim 2, wherein the corresponding genotype comprises AA.

10. The method of claim 2, wherein the corresponding genotype comprises AG.

## Patentansprüche

1. Verfahren zur Vorhersage, ob ein Patient mit feuchter AMD mit höherer Wahrscheinlichkeit von einer Behandlung mit einem hochaffinen Anti-VEGF-Antikörper profitieren wird, umfassend das Screenen einer aus dem Patienten isolierten Probe auf einen genomischen Polymorphismus im Matrix-Metalloprotease-25-Gen-(MMP25-) Allel, das rs1064875 entspricht, wobei der Patient mit höherer Wahrscheinlichkeit von dieser Behandlung profitiert, wenn der entsprechende Genotyp AA oder AG umfasst.

2. Verfahren zur Vorhersage, ob ein Patient mit feuchter AMD mit höherer Wahrscheinlichkeit von einer Behandlung mit einem Anti-VEGF-Antikörper profitieren wird, umfassend das Screenen einer aus dem Patienten isolierten Probe auf einen genomischen Polymorphismus im basalen Leucin-Zipper-Transkriptionsfaktor, ATF-ähnlichen (BATF-) Allel, das rs175714 entspricht, wobei der Patient mit höherer Wahrscheinlichkeit von dieser Behandlung profitiert, wenn der entsprechende Genotyp AA oder AG umfasst.

3. Verfahren nach einem der Ansprüche 1 oder 2, wobei der Anti-VEGF-Antikörper das gleiche Epitop bindet wie der monoklonale Anti-VEGF-Antikörper A4.6.1, der vom Hybridom ATCC® HB 10709 erzeugt wird.

4. Verfahren nach Anspruch 3, wobei der Anti-VEGF-Antikörper eine variable Schwerkettendomäne aufweist, welche die folgenden Aminosäuresequenzen der komplementaritätsbestimmenden Region (CDR) der schweren Kette umfasst: CDRH1 (GYDFTHYGMN; Seq.-ID Nr. 1), CDRH2 (WINTYTGEPTYAADFKR; Seq.-ID Nr. 2) und CDRH3 (YPYYYGTSHWYFDV; Seq.-ID Nr. 3), und eine variable Leichtkettendomäne aufweist, welche die folgenden Leichtketten-CDR-Aminosäuresequenzen umfasst: CDRL1 (SASQDISNYLN; Seq.-ID Nr. 4), CDRL2 (FTSSLHS; Seq.-ID Nr. 5) und CDRL3 (QQYSTVPWT; Seq.-ID Nr. 6).

5. Verfahren nach Anspruch 4, wobei der Anti-VEGF-Antikörper die variable Schwerkettendomäne und die variable Leichtkettendomäne von Y0317 aufweist.

6. Verfahren nach einem der Ansprüche 1 bis 3, wobei der Anti-VEGF-Antikörper Ranibizumab ist.

7. Verfahren nach Anspruch 1, wobei der entsprechende Genotyp AA umfasst.

8. Verfahren nach Anspruch 1, wobei der entsprechende Genotyp AG umfasst.

9. Verfahren nach Anspruch 2, wobei der entsprechende Genotyp AA umfasst.

10. Verfahren nach Anspruch 2, wobei der entsprechende Genotyp AG umfasst.

## Revendications

1. Procédé pour prédire si un patient souffrant d'une AMD humide a une probabilité accrue de bénéficier d'un traitement à l'aide d'un anticorps anti-VEGF à haute affinité, comprenant le criblage d'un échantillon isolé à partir dudit patient pour un polymorphisme génomique dans l'allèle du gène de la métalloprotéase matricielle 25 (MMP25) correspondant à rs1064875, dans lequel le patient a une probabilité accrue de bénéficier dudit traitement si le génotype correspondant comprend AA ou AG.

2. Procédé pour prédire si un patient souffrant d'une AMD humide a une probabilité accrue de bénéficier d'un traitement à l'aide un anticorps anti-VEGF, comprenant le criblage d'un échantillon isolé à partir dudit patient pour un polymorphisme génomique dans l'allèle de type ATF (BATF) du facteur de transcription à glissière à leucine basique correspondant à rs175714, dans lequel le patient a une probabilité accrue de bénéficier dudit traitement si le génotype correspondant comprend AA ou AG.

3. Procédé selon l'une quelconque des revendications 1 ou 2, dans lequel ledit anticorps anti-VEGF se lie au même épitope que l'anticorps anti-VEGF monoclonal A4.6.1 produit par hybridome ATCC® HB 10709.

4. Procédé selon la revendication 3, dans lequel ledit anticorps anti-VEGF a un domaine variable à chaîne lourde comprenant les séquences d'acides aminés de région déterminant la complémentarité (CDR) à chaîne lourde suivantes : CDRH1 (GYDFTHYGMN ; SEQ ID NO: 1), CDRH2 (WINTYTGEPTYAADFKR ; SEQ ID NO: 2) et CDRH3 (YPYYYGTSHWYFDV ; SEQ ID NO: 3) et un domaine variable à chaîne légère comprenant les séquences d'acides aminés CDR à chaîne légère suivantes : CDRL1 (SASQDISNYLN ; SEQ ID NO: 4), CDRL2 (FTSSLHS ; SEQ ID NO: 5) et CDRL3 (QQYSTVPWT ; SEQ ID NO: 6).

5. Procédé selon la revendication 4, dans lequel ledit anticorps anti-VEGF a le domaine variable à chaîne lourde et le domaine variable à chaîne légère de Y0317.

6. Procédé selon l'une quelconque des revendications 1 à 3, dans lequel ledit anticorps anti-VEGF est le ranibizumab.

7. Procédé selon la revendication 1, dans lequel le génotype correspondant comprend AA.

8. Procédé selon la revendication 1, dans lequel le génotype correspondant comprend AG.

9. Procédé selon la revendication 2, dans lequel le génotype correspondant comprend AA.

10. Procédé selon la revendication 2, dans lequel le génotype correspondant comprend AG.


### Sequence Listing

<110> GENENTECH, INC.
   GRAHAM, Robert
<120> GENETIC POLYMORPHISMS IN AGE-RELATED MACULAR DEGENERATION
<130> P4382R1 WO
<141> 2010-10-20
<150> US 61/253,758
   <151> 2009-10-21
<160> 6
<210> 1
   <211> 10
   <212> PRT
   <213> Artificial sequence
<220>
   <223> CDRH1
<400> 1
   Gly Tyr Asp Phe Thr His Tyr Gly Met Asn
                       5                   10
<210> 2
   <211> 17
   <212> PRT
   <213> Artificial sequence
<220>
   <223> CDRH2
<400> 2
   Trp Ile Asn Thr Tyr Thr Gly Glu Pro Thr Tyr Ala Ala Asp Phe
   1               5                   10                  15
   Lys Arg
<210> 3
   <211> 14
   <212> PRT
   <213> Artificial sequence
<220>
   <223> CDRH3
<400> 3
   Tyr Pro Tyr Tyr Tyr Gly Thr Ser His Trp Tyr Phe Asp Val
                   5                   10
<210> 4
   <211> 11
   <212> PRT
   <213> Artificial sequence
<220>
   <223> CDRL1
<400> 4
   Ser Ala Ser Gln Asp Ile Ser Asn Tyr Leu Asn
                   5                   10
<210> 5
   <211> 7
   <212> PRT
   <213> Artificial sequence
<220>
   <223> CDRL2
<400> 5
   Phe Thr Ser Ser Leu His Ser
   5
<210> 6
   <211> 9
   <212> PRT
   <213> Artificial sequence
<220>
   <223> CDRL3
<400> 6
   Gln Gln Tyr Ser Thr Val Pro Trp Thr
   5